Europäisches Patentamt
European Patent Office
Office européen des brevets

(11) Veröffentlichungsnummer: 0 384 421 A2

# EUROPÄISCHE PATENTANMELDUNG

(21) Anmeldenummer: 90103297.9

(22) Anmeldetag: 21.02.90

(51) Int. Cl.5 A61K 31/55

(30) Priorität: 23.02.89 DE 3905529

(43) Veröffentlichungstag der Anmeldung: 29.08.90 Patentblatt 90/35

(84) Benannte Vertragsstaaten: AT BE CH DE DK ES FR GB GR IT LI LU NL SE

(71) Anmelder: BOEHRINGER INGELHEIM KG
D-6507 Ingelheim am Rhein(DE)
(84) BE CH DE DK ES FR GR IT LI LU NL SE AT

Anmelder: BOEHRINGER INGELHEIM INTERNATIONAL G.M.B.H.
D-6507 Ingelheim am Rhein(DE)
(84) GB

(72) Erfinder: Muacevic, Gojko, Dr.
In der Dörrwiese 13
D-6507 Ingelheim am Rhein(DE)

## (54) Verwendung von PAF-Antagonisten zur Behandlung von pathologischen Blutgasveränderungen.

(57) Die Erfindung betrifft die Verwendung von PAF-Antagonisten zur Herstellung eines Arzneimittels zur Behandlung von pathologischen Blutgasveränderungen.

EP 0 384 421 A2

## Verwendung von PAF-Antagonisten zur Behandlung von pathologischen Blutgasveränderungen

Die Erfindung betrifft die Verwendung von PAF-Antagonisten zur Behandlung von pathologischen Blutgasveränderungen und von durch Phosphorsäureestern hervorgerufenen Vergiftungen.

Vergiftungen mit Phosphorsäureestern werden im allgemeinen durch Verabreichung hoher Dosierungen von Atropin behandelt. In vielen Fällen hat es sich als besonders vorteilhaft erwiesen, zusätzlich Cholinesterasereaktivatoren - wie z. B. PAM oder Toxogonin - mit in die Therapie einzubeziehen. Trotz der Kombination von Atropin und Cholinesterasereaktivatoren war es nicht in allen Fällen möglich, das Allgemeinbefinden der Vergifteten generell zu verbessern. Die allgemeine Reaktion bei einer Vergiftung mit Organophosphaten sind: muskarinische, nikotinische und ZNS-Effekte. Die zuvorbeschriebene Therapie mit Atropin und gegebenenfalls Cholinesterasereaktivatoren ist nicht immer erfolgreich, d.h. mit der Reversibilität der Cholinesterasehemmung allein kann die Vergiftung nicht immer antagonisiert werden.

Die Bestimmung der Partialdrucke der nachfolgend aufgeführten Blutgase sowie die Bestimmung des pH-Wertes des Blutes sind ein Indiz dafür, daß eine Behandlungsmethode bei Organophosphorvergiftungen positiv anspricht.

Überraschenderweise hat sich gezeigt, daß die Verwendung von PAF-Antagonisten - insbesondere von Web 2086 - allein oder in Kombination mit Anticholinergika die Blutgaswerte bei Phosphorsäureestervergiftungen positiv beeinflußt.

In der Tabelle I sind der pH-Wert und die Partialdrucke von $CO_2$ und $O_2$ aufgeführt. Die Ergebnisse sind Mittelwerte die aus Versuchen an 6 bis 15 Ratten je Experiment erhalten wurden. Als Phosphorsäureester wurden 20 mg/kg p.o. Methylparathion - als Suspension in 0,5 %iger Tylose, 1 ml/100 g Tier - gegeben. Die Konzentration von Atropin und Web 2086 ist in der Tabelle angegeben und bezieht sich auf mg/kg i.v. Die Reihenfolge der Applikation ist in Spalte 1 der Tabelle angegeben. Die Blutentnahme erfolgt 5 Minuten nach der letzten Substanzgabe. Die Blutgas-Messung erfolgte mit einem "Corning-Blood-Gas-Analyser 178".

| | pH | $P_{CO2}$ | $P_{O2}$ |
|---|---|---|---|
| Kontrolle<br>1. Methylparathion 20 mg/kg po 15 Min. | 7,45<br>7,08 | 38,7<br>97,7 | 103,1<br>20,6 |
| 1. Methylparathion<br>2. Atropin (0.1 mg) kurativ | 6,97 | 86,9 | 42,2 |
| 1. Atropin (0.1 mg)<br>2. Web 2086 (10) präventiv<br>3. Methylparathion | 7,25 | 73,0 | 51,7 |
| 1. Methylparathion<br>2. Web 2086 (10 mg) kurativ | 7,07 | 58,9 | 61,9 |
| 1. Atropin (1 mg) präventiv<br>2. Methylparathion | 7,43 | 40,1 | 99,7 |
| 1. Atropin (0,1 mg) präventiv<br>2. Methylparathion | 7,21 | 76,9 | 40,9 |

Zur Interpretation der Meßwerte wird auf folgende Literatur verwiesen:

1. H.V. Davenport: Säure-Basen-Regulation, Thieme, 1979
2. O. Müller-Plathe: Säure-Basen-Haushalt und Blutgase, Thieme, 1982
3. W.T. Ulmer, G. Reichel, D. Nolte u. M.S. Islam: Die Lungenfunktion, Thieme 1983
4. A.G. Karczmar: Anticholinesterase Agents, Pergamon Press, 1970
5. G.B. Koelle: Cholinesterases und Anticholinesterase Agents, Volume XV of Handbuch der experimentellen Pharmakologie, Springer Verlag, 1963

Aufgrund der pharmakologischen Befunde sind PAF-Antagonisten (PAF = platelet activating fator) zur Behandlung pathologischer Blutgasveränderungen geeignet. Hierzu zählen beispielsweise auch die respiratorische Acidose (einschließlich der akuten und chronischen Acidose) - ein klinischer Zustand, der durch Retention von Kohlendioxid gekennzeichnet ist -und die metabolische Alkalose. Die erfindungsgemäßen

Verbindungen sind auch zur Behandlung des sogenannten Apnoe Syndroms geeignet. Das Apnoe-Syndrom - auch als Schlaf-Apnoe-Syndrom bezeichnet - ist durch einen auftretenden Atemstillstand während des Schlafes gekennzeichnet. Von den erfindungsgemäß genannten Verbindungen ist das 2-Brom-4-(2-chlorphenyl)-9-methyl-6H-thieno[3,2-f]1,2,4]triazolo[4,3-a][1,4]-diazepin (Brotizolam) bei der Behandlung des Schlaf-Apnoe-Syndroms bevorzugt.

Verbindungen, die als PAF-Antagonisten (PAF = platelet activating fator) Verwendung finden, sind in der Patentliteratur und der fachspezifischen Literatur bekannt, so z.B. in Prostaglandins 35, 781 (1988). Bekannte PAF-Antagonisten sind beispielsweise Verbindungen des Hetrazepintyps wie z.B. Thienodiazepine und Benzodiazepine, wie sie in den europäischen Patentanmeldungen 176 928, 176 927, 176 929 beansprucht sind.

PAF-Antagonisten sind ebenfalls in den europäischen Patentanmeldungen 230 942, 240 899, 194 416, 254 245, und 255 028 offenbart, auf die hiermit inhaltlich Bezug genommen wird. Von besonderem Interesse sind PAF-Antagonisten des Hetrazepintyps, die als Strukturelement die allgemeine Formel I

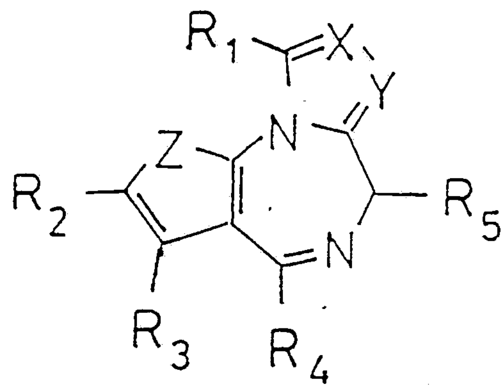

I

aufweisen.

In der allgemeinen Formel I bedeuten

X Stickstoff, C-H, C - Alkyl,

Y Stickstoff, C-H, C - Alkyl,

Z Schwefel oder den Rest -CH = CH-,

$R_1$ Wasserstoff, Halogen, Alkyl,

$R_2$ Wasserstoff, Halogen, ein Alkyl, Alkenyl- oder Alkinylrest, der durch eine funktionelle Gruppe substituiert sein kann oder eine funktionelle Gruppe,

$R_3$ Wasserstoff, Alkyl oder $R_2$ und $R_3$ bilden zusammen einen ankondensierten 5- oder 6-gliedrigen Cycloalkylrest, der als weiteren Substituenten einen Rest tragen kann, wobei dieser Halogen, ein Alkyl, Alkenyl-, Alkinylrest, der durch eine funktionelle Gruppe substituiert sein kann, oder eine funktionelle Gruppe bedeutet,

$R_4$ gegebenenfalls substituiertes Phenyl, Pyridyl,

$R_5$ Wasserstoff oder ein Alkylrest.

Heterazepine dieses Strukturtyps sind bekannt und in den europäischen Patentanmeldungen

EP-A-0.176,927, EP-A-0.176,928, EP-A-0.176.929, EP-A-0.194.416, EP-A-0.230.942, EP-A-0.240.899, EP-A-0.254.245, EP-A-0.255.028, EP-A-0.268.242, EP-A-0.279.681, EP-A-0.284.359, EP-A-0.291.594, EP-A-0.298.466, EP-A-0.315.698, EP-A- 0.342.456, EP-A-0.338 992, EP-A-0.328.924, EP-A-0.342.587, EP-A-0.338 993

beschrieben. Bezüglich der Definition der Reste wird ausdrücklich auf die oben genannten europäischen Patentanmeldungen Bezug genommen.

Bevorzugte Verbindungen der allgemeinen Formel I sind solche, worin X und Y beide Stickstoff, Z Schwefel, $R_1$ Methyl, $R_3$ Wasserstoff, $R_4$ ortho-Chlorphenyl, $R_5$ Wasserstoff und $R_2$ Halogen, bevorzugt Brom, eine über Alkylkette gebundene Säureamidgruppe, ein über eine Alkylkette gebundenes Amin. Bezüglich der Definitionen der genannten Reste wird inhaltlich auf die europäischen Patentanmeldungen EP-A-0 194.416 und 0.230.942 Bezug genommen.

Weiterhin bevorzugt sind Verbindungen der allgemeinen Formel, worin X, Y, Z, $R_1$,$R_4$ und $R_5$ wie zuvor definiert sind und $R_2$ und $R_3$ zusammen einen ankondensierten carbocyclischen Fünfring bilden, der als weiteren Substituenten eine Säureamidgruppe enthält, die gegebenenfalls über eine Alkylkette verknüpft ist oder als Substituenten eine Aminogruppe enthält, die gegebenenfalls über eine Alkylkette verknüpft ist.

Bezüglich der Definition der genannten Reste wird hiermit inhaltlich auf die europäische Patentanmeldung EP-A-0.254.245 Bezug genommen.

Als Säureamidgruppe gemäß oben genannter Definition ist auch ein Rest der allgemeinen Formel

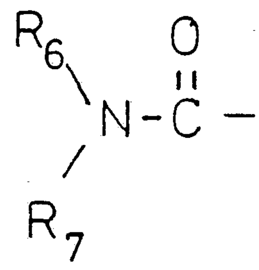

zu verstehen.
worin
$R_6$ und $R_7$, die gleich oder verschieden sein können, Wasserstoff, Phenyl, substituiertes Phenyl, eine gegebenenfalls substituierte Cycloalkylgruppe mit 3 bis 6 Kohlenstoffatomen, eine verzweigte oder unverzweigte Alkyl-, Alkenyl-oder Alkinylgruppe mit 1 bis 10 - bevorzugt 1 - 4 - Kohlenstoffatomen, die gegebenenfalls durch Halogen, Hydroxy, Nitro, Phenyl, substituiertes Phenyl, Amino, substituiertes Amino, Alkoxy 1 - 8, bevorzugt 1 - 4 substituiert sein kann;
$R_6$ oder $R_7$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom oder Stickstoff gebundener 5-, 6- oder 7-gliedriger heterocyclischer Ring;
oder
$R_6$ und $R_7$ zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann.

Als Alkylgruppen (auch soweit sie Bestandteil anderer Reste sind) werden beispielsweise genannt: Methyl, Ethyl, Propyl, iso-Propyl, Butyl, iso-Butyl, sec. Butyl, tert-Butyl, Pentyl, iso-Pentyl, Hexyl, Heptyl, Octyl, Nonyl und Dekanyl.

Als Alkenylgruppen werden beispielsweise oben genannte Alkylgruppen bezeichnet soweit sie mindestens eine Doppelbindung aufweisen, wie zum Beispiel Vinyl (soweit keine unbeständigen Enamine gebildet werden), Propenyl, iso-Propenyl, Butenyl, Pentenyl, Hexenyl.
Als Alkinylgruppen werden beispielsweise oben genannte Alkylgruppen bezeichnet, soweit sie mindestens eine Dreifachbindung aufweisen, wie zum Beispiel Propargyl, Butinyl, Pentinyl, Hexinyl.
Als Cycloalkylreste mit 3 - 6 Kohlenstoffatomen werden beispielsweise Cyclopropyl, Cyclobutyl, Cyclopentyl oder Cyclohexyl bezeichnet, die durch verzweigtes oder unverzweigtes Alkyl mit 1 bis 4 Kohlenstoffatomen, Hydroxy, und/oder Halogen substituiert sein können.

Beispielhaft für substituiertes Phenyl werden genannt:
3-Chlorphenyl, 4-Chlorphenyl, 3-Bromphenyl, 4-Bromphenyl, 4-Fluormethyl, 2-Chlorphenyl, 2-Bromphenyl, 3-Fluorphenyl, 2,3-Dichlorphenyl, 2-Methylphenyl, 4-Methylphenyl, 3-Ethylphenyl, 4-Propylphenyl, 4-Isopropylphenyl, 4-Butylphenyl, 4-tert-Butylphenyl, 4-Iso-butylphenyl, 4-Pentylphenyl, 2,4-Dimethylphenyl, 2-Trifluormethylphenyl, 3-Trifluormethylphenyl, 4-Trifluormethylphenyl, 2-Methoxyphenyl, 4-Methoxyphenyl, 3-Ethoxyphenyl, 2-Propoxyphenyl, 4-Butoxyphenyl, 2,4-Dimethoxyphenyl, 3,4,5-Trimethoxyphenyl, 2-Chlorbenzyl, 2,3-Dichlorbenzyl, 2-Methylbenzyl, 2-Trifluormethylbenzyl, 4-Methoxybenzyl, 3,4,5-Trimethoxybenzyl, 2-(2-Chlorphenyl)ethyl,

Beispiele für gegebenenfalls substituierte gesättigte oder ungesättigte heterocyclische 5-, 6- oder 7-gliedrige Ringe bzw. Heteroarylreste sind:
Pyrrol, Pyrrolin, Pyrrolidin, 2-Methylpyrrolidin, 3-Methylpyrrolidin, Piperidin - gegebenenfalls durch $C_1$-$C_4$ Alkyl ein- oder mehrfach substituiert -Piperazin, N-Methylpiperazin, N-Ethylpiperazin, N-n-Propylpiperazin, N-Benzylpiperazin, Morpholin, Thiomorpholin, Imidazol, Imidazolin, Imidazolidin, Triazol, Pyrazol, Pyrazolin, Pyrazolidin, Triazin, 1, 2, 3, 4 - Tetrazin, 1, 2, 3, 5 - Tetrazin, 1, 2, 4, 5 - Tetrazin - wobei die genannten Heterocyclen durch Alkyl mit 1 bis 4 Kohlenstoffatomen - bevorzugt Methyl - substituiert sein können;
Als heterocyclische Reste, die über ein Kohlenstoffatom verknüpft sein können, werden beispielsweise Thiophen, 2-Methylthiophen, Furan, Tetrahydrofuran, 2-Methyltetrahydrofuran, Tetrahydrofuran, 2-Hydroxymethylfuran, α-pyran, γ-Pyran, 1,3-Dioxolan, 1,2-Oxathiolan, 1,2-Oxathiepan, Tetrahydro-pyran, Thiolan, 1,3-Dithian, 1,3-Dithiolan, 1,3-Dithiolen, genannt, wobei der Heterocyclus durch $C_1$-$C_4$ Alkyl, $C_1$-$C_4$ Alkoxy, Halogen substituiert sein kann.

Als Heterocyclus im Rahmen der zuvor angegebenen Definition steht im allgemeinen auch für einen 5- bis 6-gliedrigen Ring, der als Heteroatome Sauerstoff, Schwefel und/oder Stickstoff enthalten kann, wie zum Beispiel Thienyl, Furyl, Pyridyl, Pyrimidyl, Pyrazinyl, Pyrazinyl, Chinolyl, Isochinolyl, Chinazolyl, Chinoxalyl, Thiazolyl, Benzothiazolyl, Isothiazolyl, Oxazolyl, Benzoxazolyl, Isoxazolyl, Imidazolyl, Benzimidazolyl, Pyrazolyl und Indolyl genannt.

Der Heterocyclus kann durch Halogen, Hydroxy und/oder verzweigtes oder unverzweigtes Alkyl mit 1 bis 4 Kohlenstoffatomen verzweigtes oder unverzweigtes Alkoxy mit 1 bis 4 Kohlenstoffatomen substituiert sein.

Besonders bevorzugt sind die nachfolgend genannten Verbindungen.

[4-(2-Chlorphenyl)-9-methyl-6H-thieno[3,2-f][1,2,4]triazolo-[4,3-a][1,4]diazepin-2-yl]-carbonsäuremorpholid.

[4-(2-Chlorphenyl)-9-methyl-6H-thieno[3,2-f][1,2,4]triazolo-[4,3-a][1,4]diazepin-2-yl]-carbonsäureamid

2-[4-(2-Chlorphenyl)9-methyl-6H-thieno[3,2-f][1,2,4]triazolo[4,3-a][1,4]diazepin-2-yl]-ethan-1-carbonsäurediethylamid

2-[4-(2-Chlorphenyl)-9-methyl-6H-thieno[3,2-f][1,2,4]triazolo[4,3-a][1,4]diazepin-2-yl]-ethan-1-carbonsäure-N,N-di-(2-hydroxyethyl)amid]

2-[4-(2-Chlorphenyl)-9-methyl-6H-thieno[3,2-f][1,2,4]triazolo[4,3-a][1,4]diazepin-2-yl]-ethan-1-carbonsäuremethylamid

2-[4-(2-Chlorphenyl)-9-methyl-6H-thieno[3,2-f][1,2,4]triazolo[4,3-a][1,4]diazepin-2yl]-ethan-1-carbonsäureisopropylamid

2-[4-(2-Chlorphenyl)-9-methyl-6H-thieno[3,2-f][1,2,4]triazolo[4,3-a][1,4]diazepin2-yl]-ethan-1-carbonsäuredimethylamid

2-[4-(2-Chlorphenyl)-9-methyl-6H-thieno[3,2-f][1,2,4]triazolo[4,3-a][1,4]diazepin-2-yl]-ethan-1-carbonsäure-N′-methyl-piperazid)

2-[4-(2-Chlorphenyl)-9-methyl-6H-thieno[3,2-f][1,2,4]triazolo[4,3-a][1,4]diazepin-2-yl]-ethan-1-carbonsäurepyrrolidid

2-[4-(2-Chlorphenyl)-9-methyl-6H-thieno[3,2-f][1,2,4]triazolo[4,3-a][1,4]diazepin-2-yl]-ethan-1-carbonsäurepiperidid

2-[4-(2-Chlorphenyl)-6H-thieno[3,2-f][1,2,4]triazolo[4,3-a][1,4]diazepin-2-yl]-ethan-1-carbonsäuremorpholid

2-[4-(2-Chlorphenyl)-9-brom-6H-thieno[3,2-f][1,2,4]triazolo[4,3-a][1,4]diazepin-2-yl]-ethan-1-carbonsäuremorpholid

2-[4-(2-Chlorphenyl)-9-methoxy-6H-thieno[3,2-f][1,2,4]triazolo[4,3-a][1,4]diazepin-2-yl]-ethan-1-carbonsäuremorpholid

4-(Morpholin-4yl-carbonyl)-6-(2-chlorphenyl)-11-methyl-2,3,4,5-tetrahydro-8H-[1]benzothieno[3,2-f][1,2,4]-triazolo[4,3-a][1,4]diazepin

3-(Morpholin-4-yl-carbonyl)-6-(2-chlorphenyl)-11-methyl-2,3,4,5-tetrahydro-8H-[1]benzothieno[3,2-f][1,2,4]-triazolo[4,3-a][1,4]diazepin

3-(N,N-Diethylaminocarbonyl)-6-(2-chlorphenyl)-11-methyl-2,3,4,5-tetrahydro-8H-[1]benzothieno[3,2-f][1,2,4]-triazolo[4,3-a][1,4]diazepin

4-(N,N-Diethylaminocarbonyl)-6-(2-chlorphenyl)-11-methyl-2,3,4,5-tetrahydro-8H[1]benzothieno[3,2-f][1,2,4]-triazolo[4,3-a][1,4]diazepin

4-(4-Methylpiperazinylcarbonyl)-6-(2-chlorphenyl)-11-methyl-2,3,4,5-tetrahydro-8H-[1]benzothieno[3,2-f]-[1,2,4]triazolo[4,3-a][1,4]diazepin

4-(N-Methyl-N-2-hydroxyethylaminocarbonyl)-6-(2-chlorphenyl)-11-methyl-2,3,4,5-tetrahydro-8H-[1]-benzothieno[3,2-f][1,2,4]-triazolo[4,3-a][1,4]diazepin

4-(N,N-Dimethylaminocarbonyl)-5-(2-chlorphenyl)-10-methyl-3,4-dihydro-2H,7H-cyclopenta[4,5]thieno[3,2-f]-[1,2,4] triazolo[4,3-a][1,4]diazepin

3-(N,N-Diethylaminocarbonyl-5-(2-chlorphenyl)-10-methyl-3,4-dihydro-2H,7H-cyclopenta[4,5]thieno[3,2-f]-[1,2,4]triazolo[4,3-a][1,4]diazepin

3-(N,N-Diethylaminocarbonyl-5-(2-chlorphenyl)-10-brom-3,4-dihydro-2H,7H-cyclopenta[4,5]thieno[3,2-f]-[1,2,4]triazolo[4,3-a][1,4]diazepin

4-(Morpholin-4-yl-carbonylmethylaminocarbonyl-5-(2-chlorphenyl)-10-brom-3,4-dihydro-2H,7H-cyclopenta-[4,5]thieno[3,2-f]-[1,2,4]triazolo[4,3-a][1,4]diazepin

4(Morpholin-4-yl-carbonyl)-6-(2-chlorphenyl)-11-methyl-2,3,4,5-tetrahydro-8H-[1]benzothieno[3,2-f] imidazo-[1,2-a][1,4]diazepin

4-(N,N-Diethylamino)-6-(2-chlorphenyl)-11-methyl-2,3,4,5-tetrahydro-8H-[1]benzothieno[3,2-f]imidazo[1,2-a]-

[1,4]diazepin
3-(N-Morpholinomethyl)-5-(2-chlorphenyl)-10-methyl-3,4-dihydro-2H,7H-cyclopenta[4,5]thieno[3,2-f][1,2,4]-triazolo[4,3-a][1,4]diazepin
3-(Acetoxymethyl)-5-(2-chlorphenyl)-10-methyl-3,4-dihydro-2H,7H-cyclopenta[4,5]thieno[3,2-f][1,2,4]triazolo-[4,3-a][1,4]diazepin
3-(1,2,4-Triazolyl-1-yl-methyl)-5-(2-chlorphenyl)-10-methyl-3,4-dihydro-2H,7H-cyclopenta[4,5]thieno[3,2-f]-[1,2,4]triazolo[4,3-a][1,4]diazepin
3-(N-2,6-Dimethylmorpholino-methyl)-5-(2-chlorphenyl)-10-methyl-3,4-dihydro-2H,7H-cyclopenta[4,5]thieno-[3,2-f][1,2,4]triazolo-[4,3-a][1,4]diazepin
3-Acetoxymethyl-5-(2-chlorphenyl)-3,4-dihydro-2H,7H-cyclopenta[4,5]-thieno[3,2-f][1,2,4]triazolo[4,3-a][1,4]-diazepin
4-Acetoxymethyl-6-(2-chlorphenyl)-11-methyl-2,3,4,5-tetrahydro-8H-[1]benzothieno[3,2-f]imidazo[1,2-a][1,4]-diazepin
3-Acetoxymethyl-5-(2-chlorphenyl)-10-methyl-3,4-dihydro-2H,7H-cyclopenta[4,5]thieno[3,2-f]imidazo[1,2-a]-[1,4]diazepin
3-Diethylaminomethyl-5-(2-chlorphenyl)-10-methyl-3,4-dihydro-2H,7H-cyclopenta[4,5]thieno[3,2-f][1,2,4]-triazolo[4,3-a][1,4]diazepin
4-Hydroxymethyl-6-(2-chlorphenyl)-11-methyl-2,3,4,5-tetrahydro-8H-[1]benzothieno[3,2-f][1,2,4]triazolo[4,3-a][1,4]diazepin
4-(N-Morpholinomethyl)-6-(2-chlorphenyl)-11-methyl-2,3,4,5-tetrahydro-8H-[1]benzothieno[3,2-f][1,2,4]-triazolo[4,3-a][1,4]diazepin
4-(Pyrazolyl-1-yl-methyl)-6-(2-chlorphenyl)-2,3,4,5-tetrahydro-8H-[1]benzothieno[3,2-f][1,2,4]triazolo[4,3-a]-[1,4]diazepin
4-[4,4-Dimethyl-2-oxazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin
4-[4,4-Dimethyl-2-imidazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin
4-[1,4,4-Trimethyl-2-imidazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin
4-Aminocarbonyl-6-(2-chlorphenyl)-11-methyl-2,3,4,5-tetrahydro-8H-[1]benzothieno[3,2-f][1,2,4]triazolo[4,3-a][1,4]diazepin
4-[Morpholin-4-yl-carbonyl]-6-(2-chlorphenyl)-2,3,4,5-tetrahydro-8H-[1]benzothieno[3,2-f]imidazo[1,5-a][1,4]-diazepin
4-[4,4-Dimethyl-2-thiazolin-2-yl]-6-(2-chlorphenyl)-11-methyl-2,3,4,5-tetrahydro-8H-[1]benzothieno[3,2-f]-[1,2,4]triazolo[4,3-a][1,4]diazepin
4-Amino-6-(2-chlorphenyl)-11-methyl-2,3,4,5-tetrahydro-8H-[1]benzothieno[3,2-f][1,2,4]triazolo[4,3-a][1,4]-diazepin
3-(N-Morpholinyl-methyl)-5-(2-chlorphenyl)-10-methyl-3,4-dihydro-2H,7H-cyclopenta[4,5]thieno[3,2-f]imidazo-[1,2-a][1,4]diazepin
4-(1,2,4-Triazol-1-yl-methyl)-6-(2-chlorphenyl)-11-methyl-2,3,4,5-tetrahydro-8H-[1]benzothieno[3,2-f][1,2,4]-triazol[4,3-a][1,4]diazepin
4-(1,2,4-Triazol-1-yl-methyl)-6-(2-chlorphenyl)-11-methyl-2,3,4,5-tetrahydro-8H-[1]benzothieno [3,2-f]imidazo-[1,2,-a][1,4]diazepin
4-(2-Chlorphenyl)-2-[2-(4-isobutylphenyl)ethyl]-9-methyl-6H-thieno[3,2-f][1,2,4]triazolo[4,3-a][1,4]diazepin
4-(2-Chlorphenyl)-2-[2-(4-isobutylphenyl)ethyl]-6,9-dimethyl-thieno-[3,2-f][1,2,4]triazolo[4,3-a][1,4]-diazepin.

Ganz besonders bevorzugt sind
6-(2-Chlorphenyl)-8,9-dihydro-1-methyl-8-[(4-morpholinyl)carbonyl]-4H,7H-cyclopenta[4,5]thieno[3,2-f][1,2,4]-triazolo[4,3-a][1,4]diazepin (Web 2170) (Substanz A)
4-(2-Chlorphenyl)-9-methyl-2-[3-(4-morpholinyl)-3-propanon-1-yl]-6H-thieno[3,2-f][1,2,4]triazolo[4,3-a][1,4]-diazepin (Web 2086) (Substanz B)
6-(2-Chlorphenyl-8,9-dihydro-1-methyl-8-(N,N-dipropylaminocarbonyl)4H,7H-cyclopenta[4,5]thieno[3,2-f]-[1,2,4]triazolo[4,3-a][1,4]diazepin (Web 2347) (Substanz C)
3-Morpholin-4-yl-methyl)-5-(2-chlorphenyl)-10-methyl-3,4-dihydro-2H,7H-cyclopenta[4,5]thieno[3,2-f][1-2-4]-triazolo[4,3-a][1,4]diazepin (STY 2108).
(Die Nomenklatur der Substanzen A, B, C erfolgte nach Chemical Abstracts, wobei die Numerierung des Ringsystems bei A und C in umgekehrter Reihenfolge als zuvor angegeben erfolgt).
6-(2-Chlorphenyl)-8,9-dihydro-1,4-dimethyl-8-[(4-morpholinyl)carbonyl]-4H,7H-cyclopenta[4,5]thieno[3,2-f]-[1,2,4]triazolo[4,3-a][1,4]diazepin

4-(2-Chlorphenyl)-6,9-dimethyl-2-[3-(4-morpholinyl)-3-propanon-1-yl]-6H-thieno[3,2-f][1,2,4]triazolo[4,3-a]-[1,4]diazepin

6-(2-Chlorphenyl)-8,9-dihydro-1,4-dimethyl-8-(N,N-dipropylaminocarbonyl)4H,7H-cyclopenta[4,5]thieno[3,2-f][1,2,4]triazolo[4,3-a][1,4]diazepin

Aufgrund der pharmakologischen Versuchsergebnisse erweisen sich PAF-Antagonisten - insbesondere das 4-(2-Chlorphenyl)-9-methyl-2-[3-(4-morpholinyl)-3-propanon-1-yl]-6H-thieno[3,2-f][1,2,4]triazolo[4,3-a]-[1,4-diazepin] (Web 2086) - als geeignet zur Therapie bei Phosphorsäureestervergiftungen.

Wie aus den Versuchen ersichtlich ist, zeigen sich die vorteilhaften Eigenschaften sowohl bei der Substanz allein als auch in Kombination mit Atropin oder anderen Anticholinergika.

Erfindungsgemäß können die PAF-Antagonisten allein oder als Kombination in einer Menge von 1 bis 100 mg -bevorzugt 5 bis 30 mg je Einzeldosis (intravenös) eingesetzt werden. Wird der PAF-Antagonist in Kombination mit Atropin oder einem anderen Anticholinergikum eingesetzt, so kann er gleichzeitig oder aber in getrennten Formulierungen in kurzem Zeitabstand verabreicht werden.

Da bekannt ist, daß PAF-Antagonisten selbst keine anticholinerge Wirkung besitzen, ist es bevorzugt aufgrund der Cholinesterasehemmung - Atropin oder andere Anticholinergika - bei der Therapie von Phosphorsäureestervergiftungen in Kombination mit einem PAF-Antagonisten einzusetzen.

In der nachfolgenden Tabelle sind die Säure-Base-Gleichgewichte im arteriellen Blut von Ratten vor und nach der Gabe von PAF und einem PAF-Antagonisten dargestellt.

| Substanz | Dosis ug/kg i.v. | ph | $pCO_2$ | $pO_2$ | $HCO_3$ | BE | $O_2sat$ | $TCO_2$ |
|---|---|---|---|---|---|---|---|---|
| unbehandelt | - | 7.42 | 36.4 | 95.3 | 23.4 | 0.07 | 97.3 | 24.5 |
| PAF | 10 | 7.04 | 23.0 | 115 | 6.0 | -24.0 | 95.9 | 6.8 |
| STY 2108 | 1000 | 7.40 | 39.5 | 90.4 | 24.5 | 0.42 | 96.8 | 25.8 |
| WEB 2086 | 1000 | 7.32 | 38.2 | 72.8 | 19.8 | -5.40 | 91.5 | 20.9 |
| WEB 2170 | 100 | 7.44 | 33.8 | 89.8 | 22.9 | 0.18 | 97.1 | 23.9 |

| Substanz | Dosis ug/kg i.v. | ph | $pCO_2$ | $pO_2$ | $HCO_3$ | BE | $O_2$sat | $TCO_2$ |
|---|---|---|---|---|---|---|---|---|
| unbehandelt | - | 7.47 | 35,83 | 88,50 | 26.06 | 3,58 | 96,79 | 27,18 |
| PAF | 10 | 7,12 | 20,21 | 107,93 | 6,52 | -21,23 | 95,82 | 7,15 |
| Brotizolam | Dose mg/kg p.o. | | | | | | | |
| | 10 | 7,17 | 22,22 | 104,47 | 7,88 | -18,95 | 95,48 | 8,57 |
| " | 30 | 7,37 | 35,53 | 87,42 | 20,97 | -3,32 | 94,43 | 22,05 |
| " | 100 | 7,33 | 30,60 | 87,92 | 16,38 | -7,94 | 95,18 | 17,34 |

Im Vergleich zu unbehandelten Kontrolltieren wird der pH-Wert nach Gabe von PAF (10 µg.kg i.v.) erniedrigt. Durch die untersuchten PAF-Antagonisten WEB 2086, WEB 2170 und STY 2108 wird diese pH-Wert-Erniedrigung verhindert. $CO_2$-Partialdruck und Basenüberschuß verringern sich nach Gabe von PAF. Die Abnahme wird durch Gabe der PAF-Antagonisten verhindert.

Brotizolam besitzt ebenfalls eine PAF-antagonistische Wirk-Komponente. In der gleichen Versuchsanordnung konnte die Blutgas-stabilisierende Wirkung nachgewiesen werden. Die Effekte waren dosisabhängig (10,30 und 100 mg.kg p.o.). Die durch PAF-verursachte pH Wert-Verminderung konnte durch 30 und 100 mg kg Brotizolam verhindert werden. Dies war auch bei der Abnahme des $CO_2$-Partialdruckes und für die Abnahme des Basenüberschusses der Fall.

Nachfolgend werden einige Beispiele zur Herstellung von galenischen Zübereitungen genannt, die den PAF-Antagonisten als Monosubstanz enthalten. Kombinationspräparate lassen sich in Analogie hierzu herstellen.

Beispiel 1

| Tabletten, enthaltend 20 mg Substanz B | |
|---|---|
| Zusammensetzung: | |
| Substanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45° C getrocknet. Das trockne Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht:120 mg

Beispiel 2

| Dragées, enthaltend 20 mg Substanz B | |
|---|---|
| Zusammensetzung: | |
| Substanz B | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 °C und schlägt das Granulat anschließend durch dasselbe Sieb. Nach dem Zumischen von Magnesiumstearat werden auf einer Tablettiermaschine gewölbte Drageekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.
Dragéegewicht: 130 mg

Beispiel 3

| Tabletten, enthaltend 50 mg Substanz B | |
|---|---|
| Zusammensetzung: | |
| Substanz B | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Die Substanz B, Calciumphosphat, Milchzucker und Maisstärke werden mit einer wässerigen Polyvinylpyrrolidonlösung gleichmäßig befeuchtet. Die Masse wird durch ein Sieb mit 2 mm Maschenweite gegeben, im Umlufttrockenschrank bei 50 °C getrocknet und erneut gesiebt. Nach Zumischen des Schmiermittels wird das Granulat auf einer Tablettiermaschine gepreßt.

Beispiel 4

| Kapseln, enthaltend 50 mg Substanz B | |
|---|---|
| Zusammensetzung: | |
| Substanz B | 50,0 mg |
| Maisstärke | 268,5 mg |
| Magnesiumstearat | 1,5 mg |
| | 320,0 mg |

Herstellung:

Substanz B und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

Beispiel 5

| Suppositorien, enthaltend 50 mg Substanz B | |
|---|---|
| Zusammensetzung: | |
| Substanz B | 50 mg |
| Adeps solidus | 1.650 mg |
| | 1.700 mg |

Herstellung:

Das Hartfett wird geschmolzen. Bei 40° C wird die gemahlene Wirksubstanz homogen dispergiert. Es wird auf 38° C abgekühlt und in schwach vorgekühlte Suppositorienformen ausgegossen.

Beispiel 6

| Orale Suspension, enthaltend 50 mg Substanz B pro 5 ml | |
|---|---|
| Zusammensetzung: | |
| Substanz B | 50 mg |
| Hydroxyethylcellulose | 50 mg |
| Sorbinsäue | 5 mg |
| Sorbit 70%ig | 600 mg |
| Glycerin | 200 mg |
| Aroma | 15 mg |
| Wasser ad | 5 ml |

Herstellung:

Destilliertes Wasser wird auf 70° C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz B zugegeben. Zur Entlüftung der Suspension wird unter Rühren evakuiert.

Beispiel 7

| Ampullen, enthaltend 1 mg Substanz B pro 5 ml | |
|---|---|
| Zusammensetzung: | |
| Substanz B | 1 mg |
| Natriumchlorid | 45 mg |
| Wasser für Injektionszwecke ad | 5 ml |

Herstellung:

Die Substanz B wird bei Eigen-pH in Wasser gelöst und Natriumchlorid als Isotenz zugegeben. Die erhaltene Lösung wird pyrogenfrei filtriert und das Filtrat unter aseptischen Bedingungen in Ampullen abgefüllt, die anschließend sterilisiert und zugeschmolzen werden.

Beispiel 8

| Ampullen, enthaltend 5 mg Substanz B pro 5 ml | |
|---|---|
| Zusammensetzung: | |
| Substanz B | 5 mg |
| Natriumchlorid | 45 mg |
| Wasser für Injektionszwecke ad | 5 ml |

Herstellung analog Beispiel 6.

Beispiel 9

| Ampullen, enthaltend 10 mg Substanz B pro 5 ml | |
|---|---|
| Zusammensetzung: | |
| Substanz B | 10 mg |
| Methylglucamin | 35 mg |
| Glucofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und die Substanz B unter Rühren in Lösung gebracht. Nach Zugabe der Lösungsmittel wird mit Wasser auf das Nennvolumen aufgefüllt.

Beispiel 10

| Ampullen, enthaltend 20 mg Substanz B pro 10 ml | |
|---|---|
| Zusammensetzung: | |
| Substanz B | 20,000 mg |
| Natriumchlorid | 85,000 mg |
| Natriumdihydrogenphosphat x 2 $H_2O$ | 0,925 mg |
| Dinatriumhydrogenphosphat x 2 $H_2O$ | 1,320 mg |
| 0,1 N NaOH | ad pH 6 |
| Wasser für Injektionszwecke | ad 10 ml |

Herstellung:

Die obigen Bestandteile werden in Wasser gelöst und nach Filtration wird die Lösung in sterilisierte braune Ampullen abgefüllt.

Beispiel 11

| Inhalationslösung, enthaltend 5 mg Substanz B pro 1 ml | |
|---|---|
| Zusammensetzung: | |
| Substanz B | 500 mg |
| Na-EDTA | 50 mg |
| Benzalkoniumchlorid | 25 mg |
| Natriumchlorid | 880 mg |
| Destilliertes Wasser ad | 100 mg |

Herstellung:

60 % der Wassermenge werden vorgelegt, darin nacheinander Na-EDTA, Benzalkoniumchlorid, Natriumchlorid und Substanz B klar gelöst und mit dem restlichen Wasser aufgefüllt. Die Lösung wird in 20 ml Tropfflaschen abgefüllt.

Selbstverständlich lassen sich die übrigen erfindundungsgemäßen PAF-Antagonisten in den für sie geeigneten Dosierungen in die üblichen galenischen Zubereitungen einarbeiten.

Beispiel 11

| Inhalationslösung, enthaltend 5 mg Substanz B pro 1 ml | |
|---|---|
| Zusammensetzung: | |
| Substanz B | 500 mg |
| Na-EDTA | 50 mg |
| Benzalkoniumchlorid | 25 mg |
| Natriumchlorid | 880 mg |
| Destilliertes Wasser ad | 100 mg |

Herstellung:

60 % der Wassermenge werden vorgelegt, darin nacheinander Na-EDTA, Benzalkoniumchlorid, Natriumchlorid und Substanz B klar gelöst und mit dem restlichen Wasser aufgefüllt. Die Lösung wird in 20 ml Tropfflaschen abgefüllt.

Selbstverständlich lassen sich die übrigen erfindundungsgemäßen PAF-Antagonisten in den für sie geeigneten Dosierungen in die üblichen galenischen Zubereitungen einarbeiten.

**Ansprüche**

1) Mittel zur Behandlung von pathologischen Blutgasveränderungen, dadurch gekennzeichnet, daß es wenigstens einen PAF-Antagonisten enthält.

2) Mittel nach Anspruch 1, dadurch gekennzeichnet, daß der PAF-Antagonist Web 2086, Web 2170 oder Web 2347 ist.

3) Verwendung von PAF-Antagonisten zur Herstellung eines Arzenimittels zur Behandlung von pathologischen Blutgasveränderungen.

4) Verwendung von Brotizolam zur Herstellung eines Arzneimittels zur Behandlung des Schlaf-Apnoe-Syndroms.

5) Mittel zur Behandlung von Phosphorsäureestervergiftungen, dadurch gekennzeichnet, daß es wenigstens einen PAF-Antagonisten und wenigstens ein Anticholinergikum enthält.

6) Mittel nach Anspruch 5, dadurch gekennzeichnet, daß der PAF-Antagonist Web 2086, Web 2170 oder Web 2347 ist.

7) Mittel nach Anspruch 5 oder 6, dadurch gekennzeichnet, daß das Anticholinergikum Atropin ist.

8) Verwendung von PAF-Antagonisten in Kombination mit Anticholinergika zur Herstellung eines Arzneimittels zur Behandlung von Phosphorsäureestervergiftungen.

9) Verwendung von PAF-Antagonisten zur Herstellung eines Arzneimittels zur Behandlung von Phosphorsäureestervergiftungen.

10) Verwendung nach Anspruch 8 oder 9, dadurch gekennzeichnet, daß der PAF-Antagonist Web 2086, Web 2170 oder Web 2347 ist.

11) Verwendung nach Anspruch 9, dadurch gekennzeichnet, daß das Anticholinergikum Atropin ist.